Europäisches Patentamt

European Patent Office

Office européen des brevets

(11) Veröffentlichungsnummer: **0 275 459 B1**

# EUROPÄISCHE PATENTSCHRIFT

(45) Veröffentlichungstag der Patentschrift: **27.02.91**

(51) Int. Cl.⁵: **A61F 13/14**

(21) Anmeldenummer: **87118219.2**

(22) Anmeldetag: **09.12.87**

(54) **Schultergelenkbandage.**

(30) Priorität: 18.12.86 DE 8633843 U
     12.02.87 DE 3704288

(43) Veröffentlichungstag der Anmeldung:
     **27.07.88 Patentblatt 88/30**

(45) Bekanntmachung des Hinweises auf die
     Patenterteilung:
     **27.02.91 Patentblatt 91/09**

(84) Benannte Vertragsstaaten:
     **CH DE FR GB IT LI NL**

(56) Entgegenhaltungen:
     **EP-A- 0 202 474**
     **DE-A- 2 140 981**
     **GB-A- 1 107 833**
     **US-A- 3 499 441**
     **US-A- 3 906 944**

(73) Patentinhaber: **Bauerfeind GmbH & Co.**
     **Arnoldstrasse 15**
     **D-4152 Kempen 1(DE)**

(72) Erfinder: **Scheuermann, Rainer, Dr. med.**
     **Rönner Weg 5**
     **D-2313 Raisdorf(DE)**

(74) Vertreter: **Stark, Walter, Dr.-Ing.**
     **Moerser Strasse 140**
     **D-4150 Krefeld(DE)**

## Beschreibung

Die Erfindung betrifft eine Schultergelenkbandage mit einem den Oberarm umgreifenden schlauchförmigen Abschnitt aus einem elastischen Material und einem sich an den schlauchförmigen Abschnitt anschließenden, die Schulter abdeckenden kappenförmigen Abschnitt aus einem elastischen Material.

Es ist gewünscht, zur Nachbehandlung von operativen Eingriffen am Schultergelenk oder Schultereckgelenk, bei Rotatorenmanschettenrupturen, schmerzhafter Schultersteife, bei subcapitaler Humerusfraktur, Schulterluxation oder bei einer Schultereckgelenksprengung die verletzte Schulter zu bandagieren, um die Schmerzen zu lindern und den Heilungsprozeß zu unterstützen.

Eine gattungsgemäße Schultergelenkbandage (EP 02 02 474 A 1) wird mit einem sich über den Brustkorb und einem sich über den Rücken des Patienten erstreckenden Halteband festgelegt. Die rotierende Wirkung der Haltebänder ist schwer zu beherrschen.

Der Erfindung liegt die Aufgabe zugrunde, eine Schultergelenkbandage zu schaffen, die willkürliche Außenund Innenrotation sowie die Abduktion und Elevation begünstigt.

Erfindungsgemäß wird diese Aufgabe gelöst durch einen im oberen Bereich des dem kappenförmigen Abschnitt entfernten Randes des schlauchförmigen Abschnitts an diesen angesetzten, wendelförmig um den schlauchför migen Abschnitt und an einem der Seitenränder des kappenförmigen Abschnitts entlang und sodann unter die Achselhöhle der anderen Schulter hindurch verlaufenden ersten Extensionsgurt und einen im oberen Bereich des dem kappenförmigen Abschnitt entfernten Randes des schlauchförmigen Abschnitts an diesen angesetztenm, gegenläufig zu dem ersten Extentionsgurt diesen kreuzend wendelförmig um den schlauchförmigen Abschnitt und an dem anderen Seitenrand des kappenförmigen Abschnitts entlang und sodann unter erneutem Kreuzen des ersten Extensionsgurtes über den Rücken des Patienten verlaufenden und dort mit dem ersten Extensionsgurt verbundenen zweiten Extensionsgurt.

Vorzugsweise ist vorgesehen, daß wenigstens das Material des schlauchförmigen Abschnitts längselastisch ausgebildet ist.

Eine besonders bevorzugte Ausgestaltung zeichnet sich dadurch aus, daß die beiden Extensionsgurte über ein eine Längenverstellung ermöglichendes Verschlußstück miteinander verbunden sind.

Zur Erhöhung der Bequemlichkeit ist weiter ein von dem ersten Extensionsgurt verschiebbar getragenes Polster vorgesehen.

Weiter kann vorgesehen sein, daß der schlauchförmige Abschnitt und/oder der kappenförmige Abschnitt mit die Extensionsgurte aufnehmenden Taschen versehen sind.

Eine besonders bevorzugte Ausführungsform zeichnet sich dadurch aus, daß die Extensionsgurte im Bereich ihrer auf dem Rücken des Patienten liegenden Kreuzung miteinander vernäht sind.

Um einen gezielten Druck auf das Schultereckgelenk zu bewirken, kann vorgesehen sein, daß der kappenförmige Abschnitt im Bereich des Schultereckgelenks mit einer zur Aufnahme einer Pelotte dienenden Tasche versehen ist.

Die Erfindung wird im folgenden anhand einer Zeichnung erläutert. Dabei zeigt:

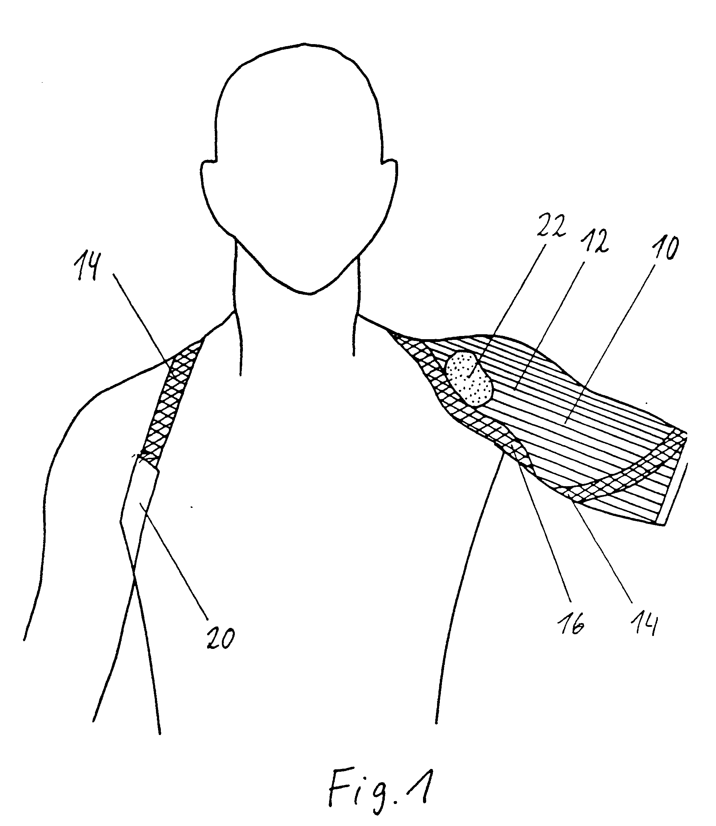

Fig. 1 die Schultergelenkbandage in einer Ansicht von vorne, und

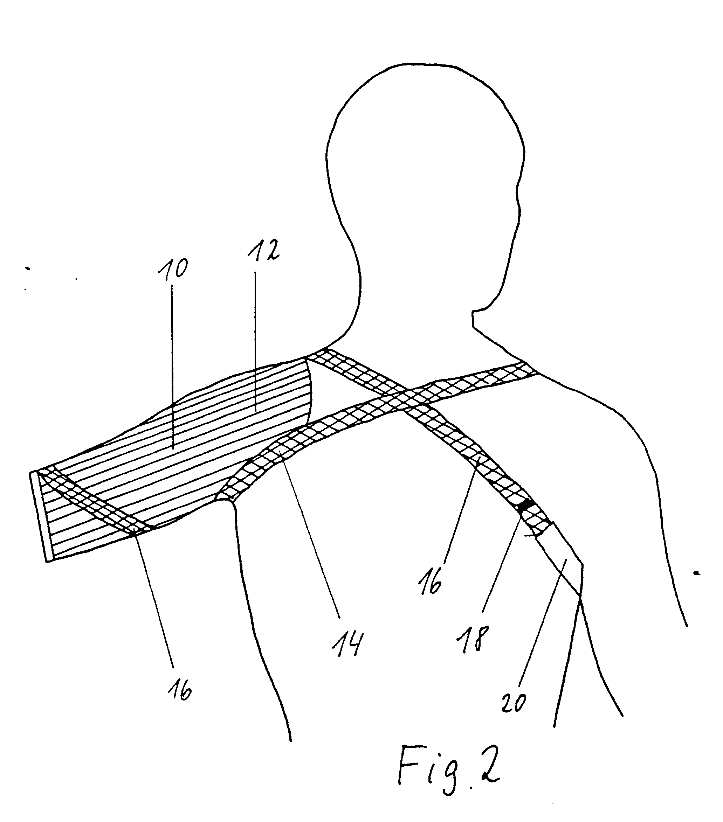

Fig. 2 die Schultergelenkbandage in einer Ansicht von hinten.

Die Schultergelenkbandage besteht aus einem den Oberarm umgreifenden schlauchförmigen Abschnitt 10 aus einem elastischen Material, einem an diesen angestetzten, vorzugsweise einstückig gestrickten, die Schulter abdeckenden kappenförmigen Abschnitt 12, der ebenfalls aus einem elastischen Material besteht, sowie zwei Extensionsgurten 14, 16. Der erste Extensionsgurt 14 ist im oberen Bereich des dem kappenförmigen Abschnitt 12 entfernten Randes, also im Bereich des Ellenbogens, an den schlauchförmigen Abschnitt 10 angesetzt und verläuft zunächst nach vorne wendelförmig um den schlauchförmigen Abschnitt 10 herum, unter der Achselhöhle der zu bandagierenden Schulter hindurch, an dem hinteren Seitenrand des kappenförmigen Abschnitts 12 entlang über den Rücken, die andere Schulter hinweg und unter der anderen Achselhöhle wieder hindurch. Der zweite Extensionsgurt 16 verläuft von dem dem kappenförmigen Abschnitt 12 abgewandten Rand des schlauchförmigen Abschnitts 10 zunächst nach hinten wendelförmig um diesen herum, sodann entlang des vorderen Randes des kappenförmigen Abschnitts 12 über die Schulter hinweg den ersten Extensionsgurt 14 kreuzend in Richtung auf die Achselhöhle der anderen Schulter. Im Bereich des Rückens sind die beiden Extensionsgurte 14, 16 mit Verschlußstücken 18 versehen, die eine Längenverstellung wenigstens eines der Extensionsgurte 14, 16 ermöglichen.

Der erste Extentionsgurt 14 trägt ein Schiebepolster 20, um Druckbeschwerden oder Wundreiben im Bereich des hier gebildeten Gegenlagers zu vermeiden.

Zeichnerisch nicht dargestellt ist ein bevorzugtes Ausführungsbeispiel, bei dem die Extensionszüge im Bereich des schlauchförmigen und/oder des kappenförmigen Abschnitts 10, 12 in Taschen geführt sind. Weiter ist zeichnerisch nicht dargestellt, daß die beiden Extensionszüge 14, 16 im Bereich der ihrer Kreuzung auf dem Rücken miteinander vernäht sein können.

Erkennbar ist dagegen eine Tasche 22, die im Bereich des Schultereckgelenks auf den kappenförmigen Abschnitt 12 aufgenäht ist. Diese dient dazu, eine Silikon-Pelotte aufzunehmen, um so gezielten Druck auf das Schultereckgelenk auszuüben.

Die vorgeschlagene Schultergelenkbandage ermöglicht ein einfaches und wirksames Bandagieren der Schulter. Die außenrotierende Wirkung des ersten Extensionsgurtes wird von dem gegenläufig angeordneten zweiten Extensionszugs 16 aufgehoben. Die willkürliche Außen-und Innenrotation sowie die Abduktion und Elevation werden durch die Extensionsgurte 14, 16 begünstigt. Der entlang des vorderen Seitenrandes des kappenförmigen Teiles 12 verlaufende zweite Extensionsgurt 16 stützt das subakrominale Nebengelenk.

Es versteht sich, daß die jeweils einzusetzende Schultergelenkbandage je nach der anatomischen Ausbildung des Schulterbereiches des jeweiligen Patienten aus einem Vorrat von Standardgrößen einzusetzen ist.

## Ansprüche

1. Schultergelenkbandage mit einem den Oberarm umgreifenden schlauchförmigen Abschnitt (10) aus einem elastischen Material und einem sich an den schlauchförmigen Abschnitt (10) anschließenden, die Schulter abdeckenden kappenförmigen Abschnitt (12) aus einem elastischen Material, gekennzeichnet durch
   - einen im oberen Bereich des dem kappenförmigen Abschnitt (12) entfernten Randes des schlauchförmigen Abschnitts (10) an diesen angesetzten, wendelförmig um den schlauchförmigen Abschnitt (10) und an einem der Seitenränder des kappenförmigen Abschnitts (12) entlang und sodann unter die Achselhöhle der anderen Schulter hindurch verlaufenden ersten Extensionsgurt (14) und
   - einen im oberen Bereich des dem kappenförmigen Abschnitt (12) entfernten Randes des schlauchförmigen Abschnitts (10) an diesen angesetzten, gegenläufig zu dem ersten Extensionsgurt (14) diesen kreuzend wendelförmig um den schlauchförmigen Abschnitt (10) und an dem anderen Seitenrand des kappenförmigen Abschnitts (12) entlang und sodann unter erneutem Kreuzen des ersten Extensionsgurts (14) über den Rücken verlaufenden und dort mit dem ersten Extensionszug (14) verbundenen zweiten Extensionsgurt (16).

2. Schultergelenkbandage nach Anspruch 1, dadurch gekennzeichnet, daß wenigstens das Material des schlauchförmigen Abschnitts (10) längselastisch ausgebildet ist.

3. Schultergelenkbandage nach Anspruch 1 oder Anspruch 2, dadurch gekennzeichnet, daß die beiden Extensionsgurte (14, 16) über einen eine Längenverstellung ermöglichendes Verschlußstück (18) miteinander verbunden sind.

4. Schultergelenkbandage nach einem der vorangehenden Ansprüche, gekennzeichnet durch einen von dem ersten Extensionsgurt verschiebbar getragenes Polster (20).

5. Schultergelenkbandage nach einem der vorangehenden Ansprüche, dadurch gekennzeichnet, daß der schlauchförmige Abschnitt (10) und/oder der kappenförmige Abschnitt (12) mit die Extensionsgurte (14, 16) aufnehmenden Taschen versehen sind.

6. Schultergelenkbandage nach einem der vorangehenden Ansprüche, dadurch gekennzeichnet, daß die Extensionsgurte (14, 16) im Bereich ihrer auf dem Rücken des Patienten liegenden Kreuzung miteinander vernäht sind.

7. Schultergelenkbandage nach einem der vorangehenden Ansprüche, dadurch gekennzeichnet, daß der kappenförmige Abschnitt (12) im Bereich des Schultereckgelenks mit einer zur Aufnahme einer Pelotte dienenden Tasche (22) versehen ist.

## Claims

1. Shoulder joint bandage, including a tubular portion (10), which surrounds the upper arm and is made of a resilient material, and a cap-like portion (12), which is connected to the tubular portion (10), covers the shoulder and is made of a resilient material, characterised by
   - a first extension strap (14), which is attached to the upper edge region of said tubular portion (10) remote from the cap-like portion (12), is wound helically around the tubular portion (10), extends along one of the lateral edges of the cap-like portion (12) and then extends beneath the armpit of the other shoulder, and

- a second extension strap (16), which is attached to the upper edge region of said tubular portion (10) remote from the cap-like portion (12), is wound helically around the tubular portion (10) in a direction opposite that of the first extension strap (14) so as to intersect with the latter, extends along the other lateral edge of the cap-like portion (12) and then extends over the back, thereby intersecting the first extension strap (14) again, and is connected there to the first extension strap (14).

2. Shoulder joint bandage according to claim 1, characterised in that at least the material of the tubular portion (10) is longitudinally resilient.

3. Shoulder joint bandage according to claim 1 or claim 2, characterised in that the two extension straps (14, 16) are interconnected through the intermediary of a closure member (18), which permits longitudinal adjustment.

4. Shoulder joint bandage according to one of the preceding claims, characterised by a cushioning pad (20), which is displaceably supported by the first extension strap.

5. Shoulder joint bandage according to one of the preceding claims, characterised in that the tubular portion (10) and/or the cap-like portion (12) are provided with cavities (22) which accommodate the extension straps (14, 16).

6. Shoulder joint bandage according to one of the preceding claims, characterised in that the extension straps (14, 16) are sewn together in the region where they intersect on the back of the patient.

7. Shoulder joint bandage according to one of the preceding claims, characterised in that, in the region of the acromion of the shoulder joint, the cap-like portion (12) is provided with a cavity (22) for the accommodation of a pad.

**Revendications**

1. Bandage articulé d'épaule, comprenant une section en forme de tuyau (10) entourant la partie supérieure du bras, composée d'un matériau élastique et une section en forme de coiffe (12), recouvrant l'épaule et se raccordant à la section en forme de tuyau (10), composée d'un matériau élastique caractérisé par

- une première sangle d'extension (14), située dans la zone supérieure du bord, éloigné de la section en forme de coiffe (12), de la section en forme de tuyau (10) et appliquée sur cette dernière, en forme d'hélice passant autour de la section en forme de tuyau (10) et le long de la section en forme de coiffe (12), puis au-dessous du creux de l'aisselle de l'autre épaule, et

- une deuxième sangle d'extension (16), située dans la zone supérieure du bord, éloigné de la section en forme de coiffe (12), de la section en forme de tuyau (10) et appliquée sur cette dernière, en forme d'hélice de sens inverse de celui de la première sangle d'extension (14), passant, en croisant cette dernière, autour de la section en forme de tuyau (10) et le long de l'autre bord latéral de la section en forme de coiffe (12), puis, en croisant à nouveau la première sangle d'extension (14), passant au-dessus du dos et y étant relié à la première sangle (14).

2. Bandage articulé d'épaule selon la revendication 1, caractérisé en ce qu'au moins le matériau de la section en forme de tuyau (10) est élastique dans le sens de la longueur.

3. Bandage articulé d'épaule selon la revendication 1 ou 2, caractérisé en ce que les deux sangles d'extension (14,16) sont reliées entre elles par l'intermédiaire d'une pièce de verrouillage (18) permettant un réglage en longueur.

4. Bandage articulé d'épaule selon l'une des revendications précédentes, caractérisé par un coussin (20) déplaçable porté par la première sangle d'extension.

5. Bandage articulé d'épaule selon l'une des revendications précédentes, caractérisé en ce que la section en forme de tuyau (10) et/ou la section en forme de coiffe (12) sont pourvues de poches recevant les sangles d'extension (14,16).

6. Bandage articulé d'épaule selon l'une des revendications précédentes, caractérisé en ce que les sangles d'extension (14,16) sont cousues entre elles dans la zone de leur croisement située sur le dos du patient.

7. Bandage articulé d'épaule selon l'une des revendications précédentes, caractérisé en ce que la section en forme de coiffe (12) est pourvue dans la zone de l'articulation de coin de l'épaule d'une poche (22) servant à recevoir une pelote.

Fig.1

Fig.2